# EUROPEAN PATENT SPECIFICATION

(11) **EP 1 063 953 B1**
(45) Date of publication and mention of the grant of the patent: **24.01.2007**
(21) Application number: 99968098.6
(22) Date of filing: 07.12.1999
(51) Int. Cl.: A61F 13/20, A61F 13/82

(54) **BODY-ATTACHABLE INTERLABIAL ARTICLES AND METHODS FOR MAKING THE SAME**
AM KÖRPER ZU BEFESTIGENDE INTERLABIALE GEGENSTÄNDE UND METHODE ZU DEREN HERSSTELLUNG
ARTICLES INTERLABIAUX SE FIXANT AU CORPS ET PROCEDE DE FABRICATION

(30) Priority: 30.12.1998 US 223483
(43) Date of publication of application: 03.01.2001
(73) Proprietor: McNEIL-PPC, Inc., Skillman, NJ 08558-9418 (US)
(72) Inventor: LICHSTEIN, Bernard, M., Elizabeth, NJ 07208 (US); LUIZZI, Joseph, Michael, Jr., Newton, PA 18940 (US)
(74) Representative: Metten, Karl-Heinz
(86) International application number: PCT/US1999/029174
(87) International publication number: WO 2000/040197

(56) References cited:
- EP-A- 0 850 628
- US-A- 5 336 208
- US-A- 5 769 091

## Description

### FIELD OF THE INVENTION

The present invention relates to absorbent sanitary protection articles that are sized and configured to fit predominantly between a woman's labia minora and/or majora and methods of making such articles.

### BACKGROUND OF THE INVENTION

Sanitary protection articles can generally be placed into three categories: external pads that are positioned against the perineum and attached either to a user's undergarment or directly to a user's body; interlabial articles that are worn between and near the labia minora and majora: and internal articles such as tampons and collection devices that are worn within the vaginal canal.

A large segment of menstruating women choose not to wear internal articles and therefore rely mainly on external pads, and more specifically on interlabial articles. External pads typically have large dimensions since they capture fluid after the fluid has exited the vagina, having potentially traveled in multiple directions along or outward from the body. After external pads capture fluids, the possibility of fluid leakage is increased by distortion, resulting from both the body's movement against the pad, and the undergarments, to which all commercial pads are attached, pulling the pad away from the body.

In an effort to reduce the failure rate of garment attached external pads, products have been invented to be attached directly to the body, see for example US Patent Number 5,658,270. However, such body adhering pads must be attached to the body along substantial portions of their body-contacting surface, to efficiently capture fluid. Interlabial articles, which are well known in the art, capture fluid closer to the site of discharge than do external pads and yet do not reside within the vaginal canal, such as a tampon does. Residing between the labia, they are much smaller and discreet than pads. A recent example of an interlabial invention is disclosed in US Patent Number 5,484,429, which describes an article comprising a fluid pick-up module, wicking fibers and a capacity module. Other inventions, e.g., US patent Numbers 5,074,855 and 5,336,208, describe devices that are designed to fit between the labia minora and the vestibule to seal and occlude the urethral meatus, for controlling female urinary incontinence. However, such devices are intended to prevent the exit of urine and not to absorb it, and other bodily fluids such as menstrual discharge.

Interlabial articles until now have depended either on friction, close fit or a garment-attaching ancillary device to enable them to be positioned to absorb liquid discharges from the body and to maintain their position during use. Friction or fit separately, or in combination, has not proved to be reliable means for retaining these articles. Attachment by ancillary devices to the undergarment encourages the interlabial article to shift and pull away from the labia during use, similar to that experienced with external pads, thereby leading to the potential of leakage and irritation.

EP 0 850 628 A1 discloses a disposable adsorbent article designed to be worn externally of the body by a user and to receive fluids discharged from the body. Said known disposable adsorbent article comprises an adsorbent core having a front portion, a central portion and a rear portion, and comprises a longitudinally oriented ridge in the central and rear portions having a slope that decreases rearwardly. The disposable adsorbent article is intended for direct attachment to the skin of the wearer and comprises an adhesive on the body facing surface.

US-A-5 769 091 relates to an urethral plug with a hollow body adapted to internally occlude the urethra. The plug functions in the urethra only to block the flow of urine.

Thus, there exists a need for an interlabial article that stays in place, is not inadvertently dislodged, and is atraumatic to wear and to remove. These requirements are the broad objectives of this invention.

### SUMMARY OF THE INVENTION

In accordance with one embodiment of the present invention, there has now been provided an absorbent interlabial article sized and configured for adequate and comfortable penetration of the space between the labia, comprising: an absorbent structure, having a proximal part, which in use faces the body and penetrates the labia, and a distal part, which in use faces opposite the body, comprising body-facing and opposite-facing outwardly disposed surfaces respectively; an adhesive located on at least a portion of the body-facing outwardly disposed surface, for releasably attaching the article to the user's body; a height for penetration of the space between the labia by the absorbent article that is adequate to capture and absorb liquid discharges from the body, a length that provides coverage of the vaginal opening and the urethral meatus and a width that provides good fit for the space between the labia.

In addition to the unique absorbent articles above, the present invention also provides a method for making an adsorbent interlabial article sized and configured for adequate and comfortable penetration of the space between the labia and comprising a height that is adequate to capture and absorb liquid discharges from the body, a length that provides coverage of the vaginal opening and the urethral meatus and a width that provides good fit for the space between the labia, comprising the following steps:
a) providing a web of absorbent material, having a central longitudinal axis;
b) covering at least part of said web with a liquid permeable material;
c) folding the web on itself along its central longitudinal axis, thereby forming a folded edge and juxtaposed sections;
d) folding the juxtaposed sections of the web outward from and coplanar with each other, along a line distal to said folded edge, thereby forming a web structure having a projection and base sections, wherein the distance between the line and the folded edge defines a projection height;
e) providing a liquid impermeable material, having a central longitudinal axis;
f) folding the liquid impermeable material on itself along its central longitudinal axis, thereby forming a folded edge and juxtaposed sections;
g) folding the juxtaposed sections of the liquid impermeable material outward from and coplanar with each other along a line distal to said folded edge, thereby forming a liquid impermeable structure having a projection and base sections, wherein the distance between the fold line and the folded edge defines a projection height;
h) aligning and attaching the web base sections to the liquid impermeable base sections, wherein the web projection and the liquid impermeable projection are outwardly disposed in opposite directions, thereby forming a absorbent structure;
i) separating the absorbent structure into a plurality of absorbent interlabial articles; and further comprising the step of applying an adhesive to at least a portion of an outwardly disposed surface of the web base sections or of an outwardly disposed surface of the web projection.

### BRIEF DESCRIPTION OF THE DRAWINGS

FIGURE 1 is a perspective view of one embodiment of the present invention.
FIGURES 2-6 illustrate multiple profiles of the absorbent interlabial article's proximal section provided by the present invention, including alternative adhesive patterns.
FIGURES 7-10 illustrate multiple cross sections of absorbent interlabial articles provided by the present invention.
FIGURES 11-13 are perspective views of multiple embodiments having differing proximal part profiles.
FIGURE 14 is a cross sectional view of an embodiment of the present invention having optional liquid accepting, transporting and storing absorbent components.
FIGURE 15 depicts an absorbent interlabial article having two lateral extensions, and adhesive regions on both the lateral extensions and a proximal part.
FIGURE 16 illustrates an embodiment of the present invention having large lateral extensions, allowing portions of the lateral extensions to wrap around a crotch section of a user's undergarments.
FIGURES 17a-17f illustrate steps of a first method for making a body-attachable interlabial article.
FIGURES 18a-18b illustrate steps of a second method for making a body-attachable interlabial article.

### DETAILED DECRIPTION OF THE INVENTION

The absorbent interlabial article of the present invention is designed to stay in place, without being inadvertently dislodged during use, be comfortable and not irritating to wear, and be easily and painlessly removed to be discarded when it is no longer needed, or when it needs to be changed. This invention effectively and efficiently absorbs liquid discharges such as those that emanate from the following: the vagina and cervix before, during and between menstrual periods, the urethral meatus and from sweat glands.

There is adhesive means incorporated into the present invention to aid in the articles secured placement to the body during use. The adhesive means are such to provide safe, non-irritaring adherence to that part of the body, to retain the device during use and to permit its safe and atraumatic removal after use.

To discuss the design, placement and use of the device it will be helpful to describe female external genital organs and their peripheral anatomical structures where the device is to be used. The perineum is the entire external genital area rearward from the anus forward to the mons pubis and laterally from the inside of one thigh to the other thigh, the mons pubis being a mound of fatty tissue, covered with pubic hair, that overlays the pubic bone. The vulva, a subsection of the perineum that encompasses the external female genitalia, includes the labia majora, which are two fatty mounds of skin covered with pubic hair that surround or cup the external genitalia. The labia majora, in turn, have inner labial surfaces and outer labial surfaces. The clitoris forms part of the anterior confluence, towards the mons pubis of the labia majora. The posterior fourchette (posterior labial commissure) forms the posterior confluence, towards the anus, of the labia majora. Anatomical openings from which liquid discharges emanate, starting from the anterior portion of the perineum rearward, are: the urethral opening through which the urethra empties urine from the body; the vaginal opening, from which vaginal discharges such as menses and other vaginal secretions empty and various sweat glands. The labia minora are soft flaps of tissue, within the labia majora, that surround the vaginal opening and clitoris. The vestibule is an area surrounding the vaginal opening and vulva.

The article can be attached to pubic hair covered parts of the perineum, such as the mons pubis and the vulva. Alternatively, the article can be attached to relatively hairless parts, such as the inner surfaces of the labia majora, the labia minora, and the inward surfaces of the thighs and the cleft between the thighs and the perineum. Multiple areas of body attachment are also provided by the present invention.

The absorbent interlabial article of the present invention comprises a proximal part, which faces the body and penetrates the labia; a distal part, which faces opposite the body; an anterior end, that faces, but preferably does not contact the clitoris in use; a posterior end, that faces, but preferably does not contact the posterior fourchette; a height that determines the degree of interlabial penetration; a length that provides coverage of the vaginal opening and the urethral meatus; and a width that provides good fit for the space between the labia.

Although, the article is intended to primarily fit between the labia, the article preferably contains lateral extensions that can have both interlabial and extralabial residence.

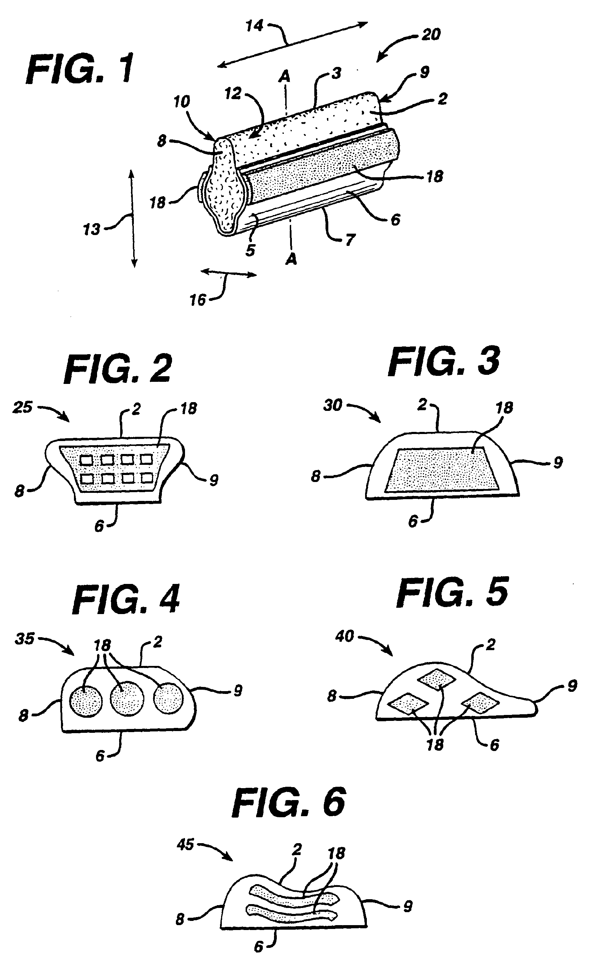

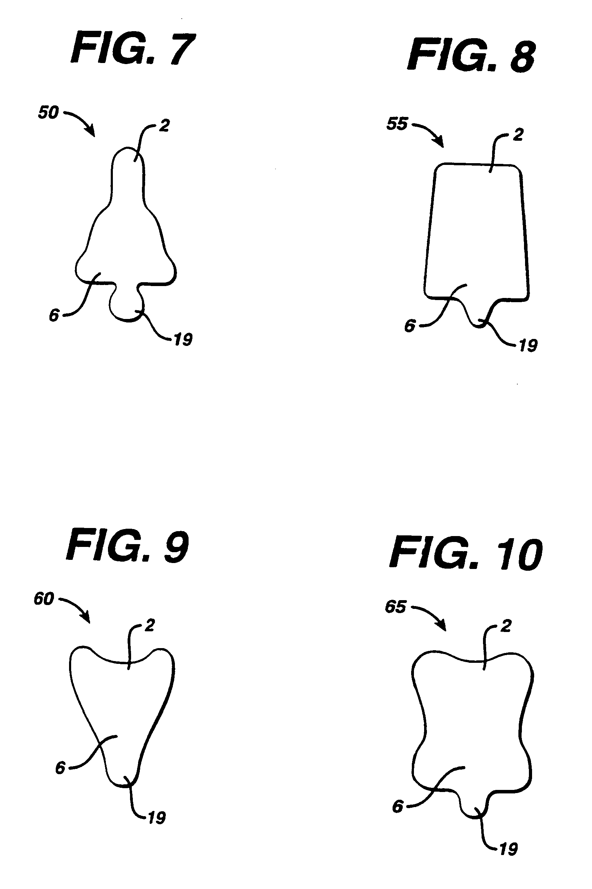

The following is a detailed description of the invention depicted in the Figures. Figure 1 shows a perspective view of a first embodiment of an absorbent article **20** of this invention, wherein this embodiment may optionally have a liquid impermeable barrier **5** to help prevent liquid from escaping absorbent article **20** onto the user's clothing. Absorbent article 20 contains absorbent material and comprises: a proximal part 2 that has an outer edge 3 which furthest penetrates the space at least between the labia majora; a distal part 6 that has an outer edge 7 and is opposite proximal part **2;** an anterior end **8** that faces but preferably does not contact the clitoris in use; a posterior end 9 that faces but preferably does not contact the posterior fourchette, two opposite lateral faces **10, 12,** located between outer edges 3 and 7, a height **13** that determines the degree of interlabial penetration, a length **14** that provides coverage of the vaginal opening and/or the urethral meatus, and a width **16** that provides good fit within space between the labia and provides good contact with at least the distal parts of the labia majora. Importantly, absorbent article **20** also comprises an adhesive **18,** preferably a body-adhering adhesive, for attaching absorbent article **20** to the body, here the labia majora and/or the vulva. Line **A-A** is also depicted in Figure 1 as a line for cross section to be referred to in Figures 7-10 forthcoming.

Height **13** should be sufficient to permit penetration of the space between the labia by absorbent article **20** to capture and absorb liquid discharges from the body, without placing excessive pressure on the labia minora and the vestibule. Height **13,** for adequate penetration, measured from the floor of the vulva outside the vulva, may be in the range from about 3 mm to about 6.5 mm, and preferably from about 6.5 mm to about 9.5 mm, depending on the anatomical dimensions of the user. Height **13** may also be greater than about 10 mm, thereby yielding a height that may be greater than that required for adequate and comfortable penetration of the space between the labia, if the excess height is permitted to be exposed outside the vulva. Such excess height is also useful as an aid for the user to grasp to insert and remove absorbent article **20.** Height **13** need not be unvarying. It may, for example, be preferably higher at posterior end **9,** to capture vaginal discharges, than at anterior end **8,** where it preferred that it does not encroach on the sensitive clitoris. It may also be the same or not the same height at anterior and posterior ends **8, 9,** respectively, and higher or lower, therebetween, than either anterior end **8** or posterior end **9.**

Length **14** may vary in the range from about 6 mm to about 75 mm, preferably from about 12 mm to about 45 mm, and most preferably from about 20 mm to about 30 mm, depending on such factors as whether absorbent article **20** is intended to cover the vaginal orifice, the urethral meatus or both, without irritatingly contacting the clitoris and posterior fourchette, and the anatomical dimensions of the user.

Length **14** may be the same as or different, in being greater or smaller, for proximal part **2** and distal part **6.** The dimension along the perimeter of the proximal part **2** will dictate the amount of coverage the article will provide for a given user. Some consumers may have needs for the article to cover both the vaginal opening and the urethral meatus, for management of cervical discharges and urine. Alternatively, an interlabial article with a proximal section having a reduced perimeter dimension, would allow for the article to remain in place for collecting cervical discharge, while providing unrestricted urination.

Width **16** may range from about 1.5 mm to as much as about 25 mm, but preferably from about 3 mm to not greater than about 12.5 mm, depending on the anatomical dimensions of the user and amount of liquid discharge it is meant to capture, and should be sufficient to fit comfortably and closely within the space between the labia to efficiently capture liquid discharges. Width 16 need not be unvarying. However it may, for example, be preferably greater or smaller at proximal part **2** than at distal part 6.

Unless otherwise noted, the number designations used in Figure 1 are understood to be the same as used throughout the remainder of the specification.

Figures 2-6 show examples, without being restricted to same, of article side views **25, 30, 35, 40, 45,** respectively, that reflect the foregoing discussion on length **14** and height **13,** where proximal part **2,** distal part **6,** anterior end **8,** posterior end **9** and one lateral face **10** are identically labeled as in Figure 1.

Figures 2-6 also show examples of alternative patterns and placement of adhesive **18** on the lateral face **10,** which is shown. Adhesive **18** may be applied in one or several places on absorbent structure **20** in the form of a continuous coating, a discontinuous coating, contiguous segments of a coating, separated segments of a coating, a patterned coating a fibrous coating and combinations thereof. Coating patterns may be such as dots, dashes, circles, symmetric, asymmetric and abstract designs and combinations thereof.

Figures 7-10 depict articles **50, 55, 60, 65,** having various cross-sectional geometries. Such cross-sectional profiles may represent anterior end **8,** posterior end **9** and any cross-section therebetween, such as taken along line **A-A** of Figure 1, of the various embodiments provided by the present invention, including those depicted in the remaining figures.

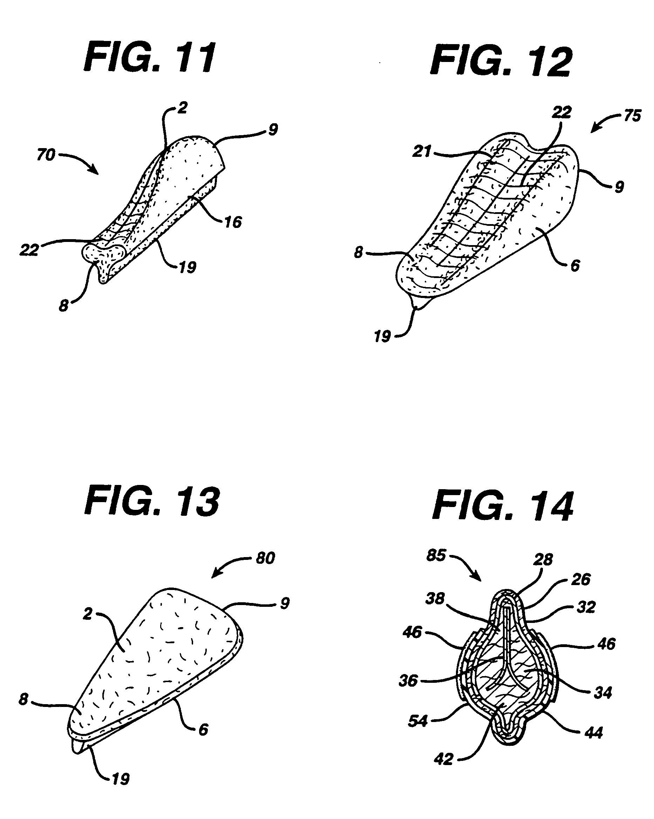

It is obvious to one skilled in the art that combinations of profiles of width and length may be employed to give optimum protection with comfort and retention of the device between the labia. The profile of proximal part **2** may differ to provide more or less contact with the female genitalia, to achieve a balance between comfort and good contact and fit for efficient capture of liquid. Figures 11 and 12 show perspective views of articles **70** and **75** having examples of such profiles, where grooves **22** on the proximal part **2** have the effect of limiting contact with the female genitalia, while providing good liquid capturing ability, and preventing the liquid from bypassing the absorbent article. Figure 13 shows an absorbent article **80,** having a flat proximal part **2** in order to provide more fluid accepting surface area, especially at the posterior end **9,** intended to be positioned proximal a user's vaginal orifice, without creating uncomfortable bulk at the anterior end **8,** intended to be positioned proximal a user's urethral meatus.

Figure 14 depicts an absorbent article **85** in a cross sectional view, illustrating liquid accepting, transporting and storing absorbent components that may exist. Absorbent article **85** has a body-facing and contacting surface **26** and an opposite-facing surface **54.** Absorbent article **85** herein comprises, sequentially, an absorbent core **34** and optionally the following: a liquid permeable cover **32;** a transfer layer **28,** to move liquid directly across to absorbent core **34;** a wicking means **36** to move liquid downward from a proximal part **38** toward a distal part **42;** and a liquid barrier **44.** Also shown, is an adhesive means **46,** for attaching the article directly to a user's body. A release paper (not shown) to protect the adhesive **46** before the product is used can optionally be employed.

Wicking means **36** may be made of materials having good wicking characteristics such as tissue, vertically oriented fibrous tow constructions, sphagnum moss and certain synthetic fiber webs such as polypropylene that have been treated by any number of means to make them hydrophilic and having the appropriate density and/or wicking enhancing structure to promote wicking away from the user's body and throughout the

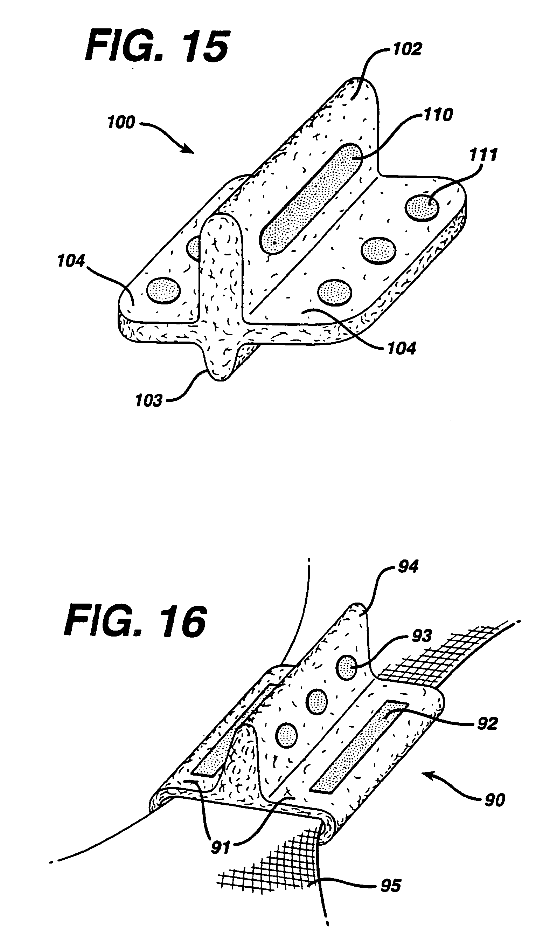

Figure 15 depicts an absorbent article of the present invention with two lateral extensions. Absorbent article **100** is shown with adhesive regions **110** and **111** located on both a proximal part **102** and the lateral extensions **104.** Adhesive regions could also be limited to either the proximal part or the lateral extensions. Optional finger-grasping member **103** is also shown. Figure 15 illustrates a single structure that can provide both liquid absorbency, and a barrier to fluid leakage. One method of forming such a single structure is by molding the article out of a foam material, and then manipulating the material and / or molding process, such as by varying heat and / or pressure, to form both open celled and closed celled surfaces.

The absorbent article of the present invention preferably has at least one lateral extension, which can extend from between the labia to outside the labia (extralabial). Preferably the article contains two lateral extensions outwardly deployed from a first and second distal edge. The lateral extensions can also contain adhesive on portions of an outwardly disposed surface directed towards a user's body. Adhesives and their techniques of application, that are useful for extralabial surfaces, such as the mons pubis, outer labial surfaces and insides of the thighs are described in commonly assigned US 5,658,270, herein incorporated by reference.

The lateral extensions are preferably constructed of material, which is inherently extensible, or can be manipulated to provide extensibility, allowing the lateral extensions to adhere to the inner thighs, and maintain the adherence during normal activity. The materials can be made extensible by performing a mechanical operation, such as pleating, corrugating, or ring rolling. In addition, the material can be perforated or slit. The perforations or slits can vary in geometry and size, thereby providing extensibility in multiple directions if needed. The materials can also be inherently stretchable, such as polyethylene blended films available from Exxon, particularly film EXX-7. A more detailed disclosure of extensible articles, and methods of making such, is contained in US Patent Number 5,824,004, herein incorporated by reference.

Also contemplated within the present invention is for the lateral extensions to be sized and configured for wrapping around, and attaching to, a crotch portion of a user's undergarment. This embodiment, as shown in Figure 16, provides adhesive located on an outwardly disposed surface of the lateral extensions directed away from the body, in order for the adhesive to adhere to the outer surface of the user's undergarment. Absorbent article **90** is shown with lateral extensions **91** wrapped around a crotch portion **95** of an undergarment. There are adhesive regions **93** located on proximal part **94,** as well as adhesive regions **92** located on the lateral extensions **91.**

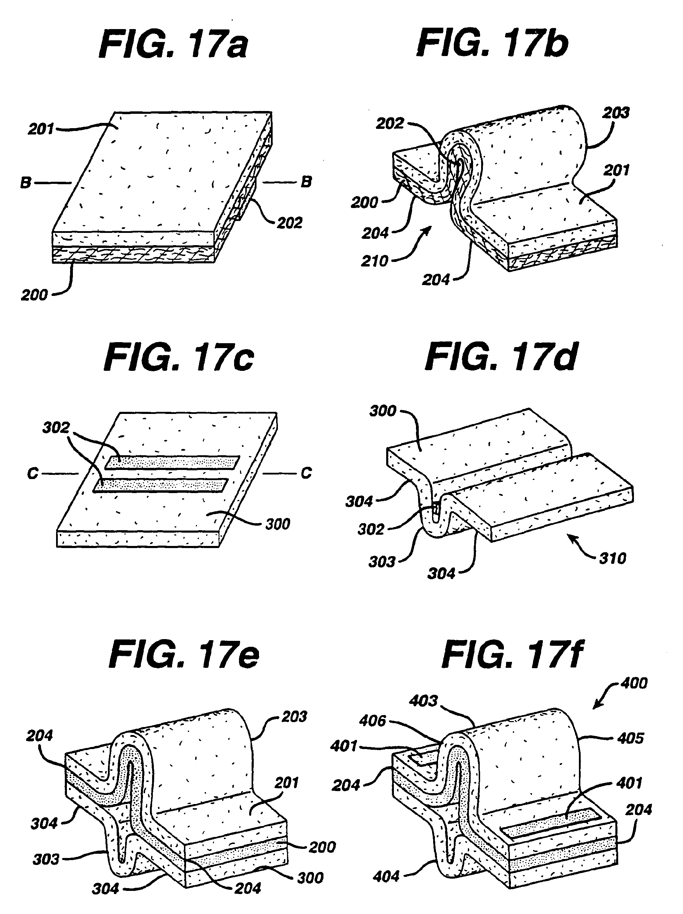

Figures 17a-17f illustrate steps included in a preferred method of making a body-adhering absorbent interlabial article as provided by the present invention. Figure 17a depicts a section of an absorbent web **200,** with optional fluid permeable cover material **201,** and optional adhesive means **202** to provide stability to a web projection formed by folding the web on itself. The absorbent web **200** is folded on itself along the web central axis **B-B,** as shown in Figure 17a, to form a web structure **210** as shown in Figure 17b, having a web projection **203,** and web base sections **204.** Similar folding steps are then followed with a liquid impermeable material, wherein the liquid impermeable material is preferably gas permeable, acting as a barrier to prevent fluid from staining the user's body or undergarments. Figure 17c shows a section of a liquid impermeable liquid impermeable material **300,** with optional adhesive means **302** to provide stability to a liquid impermeable projection formed by folding the liquid impermeable material on itself. The liquid impermeable material **300** is folded on itself along the liquid impermeable material central axis **C-C,** as shown in Figure 17c, to form a liquid impermeable structure **310** as shown in Figure 17d, having a liquid impermeable projection **303,** and liquid impermeable base sections **304.** The web structure **210** and the liquid impermeable structure **310** are then attached to one another. Figure 17e depicts the web structure **210** and the liquid impermeable structure **310,** co-extensively aligned, and attached, wherein the web projection **203** and the liquid impermeable projection **303** are outwardly deployed in opposite directions to one another. Optionally, body-adhering adhesive can be applied to a portion of the web structure.

Figure 17f illustrates a finished body-adhering absorbent interlabial article **400** made from the above steps, having adhesive **401** on body-contacting surfaces of the web base sections **204,** proximal section **403,** distal section **404,** anterior end **405,** and posterior end **406.**

The absorbent web used in the method as described above in Figures 17a-17f can be a single layer or multiple layers comprising suitable absorbent material as disclosed in the forthcoming sections of the specification. Likewise, the liquid impermeable material can be constructed with a number of materials and in a number of configurations, all of which are expanded upon below.

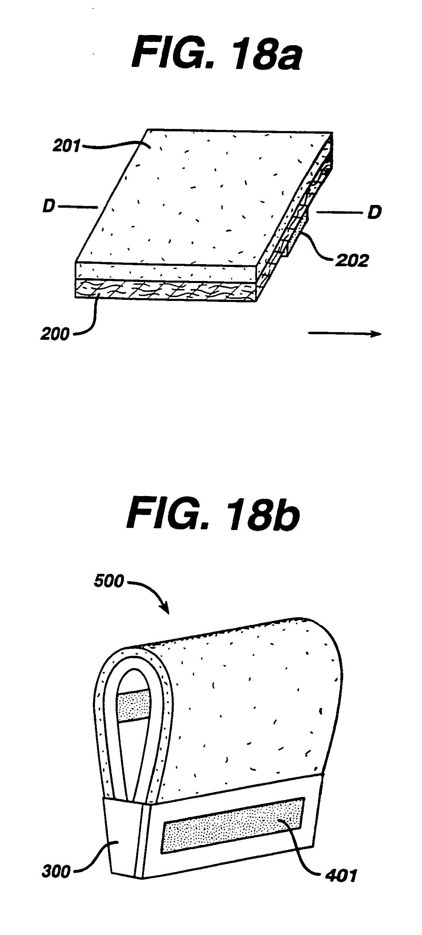

Figures 18a and 18b depict a second method of making a body-adhering absorbent interlabial article as provided by the present invention. Figure 18a shows a section of an absorbent web **200,** with optional fluid permeable cover material **201,** and optional adhesive means **202** to provide stability to a web projection formed by folding the web on itself. The web is folded on itself along the web central axis **D-D,** as shown in Figure 18a, to form a web folded edge and juxtaposed web sections. A liquid impermeable material is then wrapped around the juxtaposed web sections, totally encasing the web sections. Adhesive is then applied to a part of the liquid impermeable material, on its outward surface. Figure 18b illustrates a finished body-adhering absorbent interlabial article **500** made from the above steps, having adhesive **401** on the liquid impermeable material **300.**

The adhesive choice for embodiments of this invention depends on the nature, condition and sensitivity of the anatomical surface to which the article is to be adhered. For example, the composition and properties of adhesives that adhere to predominantly hair-containing surfaces may be different from those that adhere to relatively hairless surfaces.

Additionally, adhesives that are meant to adhere to sensitive areas such as the interlabial surfaces (associated with the labia minora and labia majora), the vestibule and to relatively less hairy internal surfaces of the vulva, will have different requirements by being more sensitive to pressure, rubbing and chafing, as well as often being moist. Those skilled in the art would readily appreciate that more than one type of adhesive, and combinations or mixtures of these adhesives, can suitable be used on the articles of the present invention.

Preferred adhesives and their formulations are those that have no or relatively low irritation potential and no sensitization potential. So called "hypoallergenic" adhesives are preferred for having little or no potentially irritating components such as certain monomers, low molecular weight components and additives such as some antioxidants, plasticizers and stabilizers.

Multiple classes of adhesives are useful in the present invention, including but not limited to solution adhesives emulsion adhesives, warm melt adhesives, and hot melt adhesives. The adhesives can be either hydrophilic or hydrophobic. A representative, non-limiting list of adhesives includes adhesives based on the following: emulsion or solvent-bome adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)ₓ wherein block A is a polyvinylarene block, block B is a poly(monoalkenyl) block, x denotes the number of polymeric arms, and wherein x is an integer greater than or equal to one; and hot melt adhesive based on olefin polymers and copolymers wherein the olefin polymer is a terpolymer of ethylene and a co-monomer, such as vinyl acetate, acrylic acid, methacrylic acid, ethyl acrylate, methyl acrylate, n-butyl acrylate vinyl silane or maleic anhydride.

Suitable block A polyvinylarenes include, but are not limited to polystyrene, polyalpha-methylstyrene, polyvinyltoluene, and combinations thereof. Suitable block B poly(monoalkenyl) blocks include, but are not limited to conjugated diene elastomers, such as for example polybutadiene or polyisoprene or hydrogenated elastomers, such as ethylene butylene or ethylene propylene or polyisobutylene, or combinations thereof. Commercial examples of block copolymers include KRATON brand elastomers from Shell Chemical Company, VECTOR brand elastomers from Dexco, SOLPRENE brand elastomers from Enichem, and STEREON brand elastomers from Firestone Tire & Rubber Co. Commercial examples of olefin polymers and copolymers include ATEVA brand polymers from AT plastics, NUCREL brand polymers from DuPont, and ESCOR brand polymers from Exxon Chemical.

Additional adhesives useful in the present invention are polysiloxane adhesives, described in US 5,618,281; pressure sensitive adhesives that are used to adhere ostomy appliances and certain wound care devices to the body, having formulations such as mixtures of gelatin, sodium carboxymethyl cellulose and polycarbons such as polyisobutylene, e.g., that described in US 4,795,449, and sold by Hollister, Inc. under the name of HOLLIHESIVE; hydrocolloids, containing, e.g., about 70% of a nitrile adhesive, as well as VISTANEX, from Exxon Corp., and pectin; hydrophilic hydrogel adhesive materials, e.g., PROMEON sold by Promeon Division of Medtronic, Inc., as described in US 5,074,855; and poly 2-hydroxvethylmethyl methacrylate, plasticized with polyethylene glycol, other plasticizers being propylene glycol, polypropylene glycol, glycerin and water, as described in US 5,336,208.

The preferred gentle (atraumatic) adhesive formulations for the present invention are "gel-like" in nature, falling within these formulations are the hydrogel and oil block copolymer based adhesives, such as those disclosed in US Patent Numbers 4,833,193; 5,559,165; and 5,074.855. The lower modulus of these gel-like adhesives will allow them to undergo greater deformation. Thusly, they can more easily encapsulate pubic hair and conform to the anatomical surfaces in the perineal region, assuring satisfactory bond strength when attached to the body. As shearing and bending forces are applied to the adhesive bond layer, during the wearing of the article, a sufficiently thick coating will allow deformation, thus limiting fracture forces that would lead the article's detachment. Upon removal, the lower adhesive strength of the adhesive gels, combined with the lubricious nature of the high oil content, will allow these adhesive gels to be removed from the skin painlessly.

The coating thickness, tack, and peel force properties of adhesives are defined below to achieve the required balance required for adequate, but not aggressive, adhesive attachment and easy, painless and otherwise atraumatic removal and reapplication to the same anatomic areas. A range of coating weights useful in this invention is from about 1.5 to about 2,000 milligrams / square centimeter, with a preferred range of from about 190 about 1,600 milligrams / square centimeter, and a most preferred range of about 300 to about 1,100 milligrams / square centimeter.

Tack values of adhesives useful in the present invention, as measured according to the standardized test method ASTM D-2979, are preferably from about 100 to about 500 grams, more preferably from about 150 to about 300 grams, and most preferably from about 175 to 250 grams. The above tack values are based on an adhesive coating thickness as described heretofore.

Peel force values of adhesives useful in the present invention, as measured according to the standardized test method PSTC-1 180 degrees peel test (Pressure Sensitive Tape Council), are preferably less than about 325 grams / centimeter width, more preferably less than about 150 grams / centimeter width, and most preferably less than about 100 grams centimeter width.

Techniques used for applying the adhesives to the article include, but are not limited to slot coating, spraying, knife coating, extrusion coating, and transfer coating, such as screen by screen and gravure. The adhesives may also be foamed prior to application, such as by using commercially available equipment from the Nordson Corporation.

Adhesives may be coated in continuous or in discrete patterns from emulsion or solution directly onto the product substrate or onto a release substrate to be subsequently transferred onto the article. If the adhesive is coated onto an article region that is meant to accept and absorb liquid, then it is preferred that the adhesive be applied as a discontinuous or fiberized coating or porous film, thereby creating voids; or that a continuous adhesive coating be hydrophilic and water penetrable by virtue of having a hydrophilic component polymer of the adhesive or suitable hydrophilic additive.

The absorbent material useful in the present invention may comprise either simple or complex absorbent structures that accept, transfer, distribute, store and retain fluid as well as prevent fluid from exiting the absorbent product. The absorbent material may be composed of one or more layers of like, or dislike elemental features. The absorbent material may be a simple absorbent such as woodpulp, which may contain stabilizing components such as synthetic fibers that are used as such to form a bridging matrix; or by being thermobondable and fused to themselves and to the woodpulp, to form a dimension stabilizing structure.

The synthetic fibers may be either hydrophilic, such as rayon, or hydrophobic such as polypropylene and polyester. The synthetic fibers may be made more wettable by treatment with a wetting agent such as a surfactant, by caustic etching of fibers such as those made of polyester, by incorporating wettable polymers such as polyethylene oxide or polyvinyl alcohol within the fiber polymer formulation, by grafting wettable reactants to the fiber surface and by exposing the fiber to corona discharge. The peripheral profile of synthetic fibers may be of any shape. The synthetic fibers may also contain grooves, channels or bores; and may be pitted or perforated. The absorbent material may also contain auxiliary absorbents such as rayon or cotton fibers, sphagnum moss and superabsorbent fibers or particles. The amount of superabsorbent material contained by the article can range from about 0.1 grams to about 5 grams, more preferably from about 0.2 grams to about 2.5 grams, and most preferably from about 0.3 grams to about grams.

Absorbents such as sphagnum moss, in board or in compressed layer form, may additionally function as compression or deformation resisting structures or to help maintain the shape of the absorbent structure in production and in use. Absorbents in board form may be made flexible and conforming by tenderizing by means such as passing the board through corrugating, embossing or perforating processes. The woodpulp itself may also be at least in part comprised of any of wet crosslinked, dry crosslinked, chemically stiffened or curly fibers. The synthetic fibers and auxiliary absorbents may be present homogeneously throughout the absorbent material, in discrete layers or in continuous or discontinuous concentration gradients. The absorbent material may also contain foam in the form of layers or particles, the foam being either hydrophobic or hydrophilic, depending on its place and function in the product, e.g., absorbing, cushioning, deformation resisting and compression resisting. The absorbent material may be uncompressed, compressed, or otherwise densified, at least in part. Compression and densification may be homogeneous throughout the absorbent material or in discrete layers or in continuous or discontinuous gradients of density.

A liquid permeable cover material may optionally be used to encase portions of the absorbent material. The liquid permeable material may be made of at least one layer of any perforate material penetrable by liquids, including woven or knitted, but more commonly nonwoven, fabrics, non reticulated open cell foam, perforate films or combinations thereof. The nonwoven fabrics include any of those made of staple fibers, continuous filaments, the staple fibers having included among them natural fibers such as cotton, jute, ramie, woodpulp and kenaf and the synthetic fibers having included among them, rayon, polyolefins such as polyethylene, polypropylene, polyesters, polyamides, polyacrylonitriles, polyvinyl esters and multicomponent fibers, the multicomponent fibers being made of a low melting polymer that is at least in part exposed on the fiber's surface, and a high melting polymer. The nonwoven fabrics may be created from the fibers and/or filaments by any of the known fabric forming processes, where applicable, such as: web consolidation and bonding processes, e.g., by wetlaying or drylaying to form a random web or by drylaying to be converted to a carded web, either web to be bonded with chemical binders or by thermal means such as through-air bonding; and by direct fabric forming, e.g., by hydroentangling, spunbonding or meltblowing.

The perforate films have perforations that may be of two or three dimensions in profile through the thickness of the film, the films being made of polymers that include polyethylenes, polypropylenes, polyurethanes, polyamides, copolymers of ethylene and vinyl acetate and the like and combinations thereof. If the fabrics or films are hydrophobic and need to be made wettable they may be made wettable, to varying degrees, e.g., by treating the film with a surfactant, by exposure to corona discharge, by grafting the film with wettable reactants, by caustic etching of films such as those made of polyester, and by incorporating wettable polymers such as polypropylene oxide and polyvinyl alcohol in the polymeric formulation used to make the fiber or film.

In an effort to improve the fluid management properties of the absorbent interlabial article, an optional transfer layer (acquisition layer) can be added, which is usually located between the absorbent material and the liquid permeable cover material. However, the transfer layer may be located elsewhere, e.g., within or below the absorbent material. Transfer layers can provide many functions, including but not limited to, wicking fluid to available absorbent material, acting as a one-way valve to prevent captured fluid from squeezing out, and providing resiliency to the article to prevent it from collapsing, especially when occupied with fluids.

Materials useful in transfer layers by be comprised of relatively less hydrophilic materials and structures, than is contained in the absorbent material, such as webs of meltblown polypropylene or polyester fibers. Such webs may also contain woodpulp entrained within. The transfer layer may also be comprised of low density, highloft nonwoven webs comprised of woodpulp and synthetic fibers such as polyethylene, polypropylene, polyester, polyacrylonitrile and polyamide and multicomponent fibers described above. Such highloft webs may be bonded with chemical binders or by thermal means such as through-air bonding and thermal embossing. Transfer layer may also be comprised of a perforate film, whose profile through the thickness of the film is either two-dimensional or preferably three-dimensional.

Multiple transfer layers may also be employed, as a means for improving the article's ability to manage fluid and for providing structurally stability. US Patent Number 5,752,945 ('945) discloses an absorbent article having a transfer sheet, wherein the transfer sheet has at least two layers. Patent '945 discloses that a first layer can provide wet and dry resilience and liquid holding capacity, while the second layer can provide lateral wicking or flow paths in order to more uniformly distribute liquid across an absorbent core.

The interlabial article may comprise additional materials to encase portions of the absorbent material. In particular, the distal section of the article can contain a liquid impermeable material as a backsheet, to help prevent the captured fluid from transferring to a user's body or clothing. The liquid impermeable backsheet may also contain portions of adhesive on its outwardly disposed surface, whereby a user can optionally attach the article to their undergarments.

The liquid impermeable backsheet can be of any flexible material that prevents the transfer through it of liquid but does not necessarily prevent the passages of gases. Commonly used materials are polyethylene or polypropylene films.

Other materials that may be used as the liquid impermeable backsheets are made from those selected from films of polyesters, polyamides, ethylene vinyl acetate, polyvinyl chloride, polyvinylidene chloride, cellophane, nitrocellulose and cellulose acetate. Coextruded and laminated combinations of the foregoing, wherein such combinations are permitted by the chemical and physical properties of the film, may be used. Liquid impermeable reticulated foams and repellent treated papers may also be used.

Films that are barriers to liquids, but permit gases to transpire, i.e., "breathable barriers", may be used. These may be selected from polyurethane films and from microporous films in which microporosity is created by ionizing radiation or by leaching out of soluble inclusions using aqueous or nonaqueous solvents. Single or multiple layers of permeable films, fabrics and combinations thereof, that provide a tortuous path, and/or whose surface characteristics provide a liquid repellent surface to the penetration of liquids may also be used to provide such breathable barriers.

There are a number of techniques useful to assembly all or the above mentioned elements into the final article, including but not limited to by heat sealing, by use of hook and fastener technology, by use of construction adhesives, by use of ultrasonics, Preferably, construction adhesives are used in adhering the elements together. A representative, non-limiting list of materials useful as the construction adhesive includes acrylics; starch based hot melts; adhesives based on block copolymers of vinyl aromatic hydrocarbon and one or more conjugated diene or hydrogenated aliphatic blocks; polylactic acids; hot melts based on polyolefins such as amorphous polyalphaolefins which may consist of one ore more of the following monomers: propylene, ehtylene, butane, and hexene; hot melts based on low density polyethylene or low density polyethylene copolymers including ethylene vinyl acetate, methyl acrylate, n-butyl acrylate, and acrylic acid.

The entire absorbent interlabial article may be made of simple combinations or composites of materials that provide both liquid absorbency and liquid impermeability.

## Claims

1. An absorbent interlabial article (20; 50; 55; 60; 65; 70; 75; 80; 85; 90; 100; 400; 500) sized and configured for adequate and comfortable penetration of the space between the labia, comprising: an absorbent structure, having a proximal part (2; 38; 94; 102), which in use faces the body and penetrates the labia, and a distal part (6; 42), which in use faces opposite the body, comprising body-facing and opposite-facing outwardly disposed surfaces (10, 12, 26, 54) respectively; an adhesive (18; 46; 401) located on at least a portion of the body-facing outwardly disposed surface (10; 26), for releasably attaching the article to the user's body; a height (13) for penetration of the space between the labia by the absorbent article (20) that is adequate to capture and absorb liquid discharges from the body, a length (14) that provides coverage of the vaginal opening and the urethral meatus and a width (16) that provides good fit for the space between the labia.

2. The article as recited in claim 1 wherein the height (13) is in the range from about 3 mm to about 6,5 mm.

3. The article as recited in claim 1 wherein the height (13) is in the range from about 6,5 mm to about 9,5 mm.

4. The article as recited in claim 1 wherein the opposite-facing outwardly disposed surface comprises a liquid impermeable backsheet.

5. The article as recited in claim 4 wherein the liquid impermeable backsheet is gas permeable.

6. The article as recited in claim 1 wherein the body-facing outwardly disposed surface comprises a liquid permeable cover (32).

7. The article as recited in claim 1 wherein a coating weight of the adhesive (18; 46; 401) is from about 1.5 to about 2,000 milligrams/square centimeter.

8. The article as recited in claim 1 wherein the adhesive (18; 46; 401) is selected from the group consisting of solution adhesives, emulsion adhesives, warm melt adhesives, hot melt adhesives and foamed adhesives.

9. The article as recited in claim 1 wherein the adhesive (18; 46; 401) is hydropholic.

10. A method for making an absorbent interlabial article sized and configured for adequate and comfortable penetration of the space between the labia and comprising a height that is adequate to capture and absorb liquid discharges from the body, a length (14) that provides coverage of the vaginal opening and the urethral meatus and a width (16) that provides good fit for the space between the labia, comprising the following steps:
a) providing a web of absorbent material, having a central longitudinal axis;
b) covering at least part of said web with a liquid permeable material;
c) folding the web on itself along its central longitudinal axis, thereby forming a folded edge and juxtaposed sections;
d) folding the juxtaposed sections of the web outward from and coplanar with each other, along a line distal to said folded edge, thereby forming a web structure having a projection and base sections, wherein the distance between the line and the folded edge defines a projection height;
e) providing a liquid impermeable material, having a central longitudinal axis;
f) folding the liquid impermeable material on itself along its central longitudinal axis, thereby forming a folded edge and juxtaposed sections;
g) folding juxtaposed sections of the liquid impermeable material outward from and coplanar with each other along a line distal to said folded edge, thereby forming a liquid impermeable structure having a projection and base sections, wherein the distance between the fold line and the folded edge defines a projection height;
h) aligning and attaching the web base sections to the liquid impermeable base sections, wherein the web projection and the liquid impermeable projection are outwardly disposed in opposite directions, thereby forming an absorbent structure;
i) separating the absorbent structure into a plurality of absorbent interlabial articles; and further comprising the step of applying an adhesive to at least a portion of an outwardly disposed surface of the web base sections or of an outwardly disposed surface of the web projection.

## Patentansprüche

1. Ein absorptionsfähiger interlabialer Gegenstand (20; 50; 55; 60; 65; 70; 75; 80; 85; 90; 100; 400; 500), der so groß und so gestaltet ist, daß er in geeigneter und komfortabler Weise in den Raum zwischen den Schamlippen eindringt, umfassend: eine absorptionsfähige Struktur mit einem proximalen Teil (2; 38; 94; 102), der bei Gebrauch zum Körper hin weist und zwischen die Schamlippen eindringt, und einem distalen Teil (6; 42), der bei Gebrauch vom Körper weg weist, die zum Körper hin weisende bzw. in die entgegengesetzte Richtung weisende, außen angeordnete Flächen (10, 12, 26, 54) umfassen; einen Klebstoff (18; 46; 401), der auf zumindest einem Abschnitt der zum Körper hin weisenden, außen angeordneten Fläche (10; 26) befindlich ist, zum lösbaren Befestigen des Gegenstandes am Körper der Nutzerin; eine Höhe (13) für das Eindringen des absorptionsfähigen Gegenstandes (20) in den Raum zwischen den Schamlippen, die geeignet ist, um flüssige Ausscheidungen aus dem Körper aufzufangen und zu absorbieren, eine Länge (14), die eine Abdeckung der Vaginaöffnung und der Harnröhrenöffnung gewährleistet, und eine Breite (16), die einen guten Sitz im Raum zwischen den Schamlippen gewährleistet.

2. Der Gegenstand nach Anspruch 1, wobei die Höhe (13) im Bereich von ca. 3 mm bis ca. 6,5 mm liegt.

3. Der Gegenstand nach Anspruch 1, wobei die Höhe (13) im Bereich von ca. 6,5 mm bis ca. 9,5 mm liegt.

4. Der Gegenstand nach Anspruch 1, wobei die in die entgegengesetzte Richtung weisende, außen angeordnete Fläche eine flüssigkeitsundurchlässige rückwärtige Schicht umfaßt.

5. Der Gegenstand nach Anspruch 4, wobei die flüssigkeitsundurchlässige rückwärtige Schicht gasdurchlässig ist.

6. Der Gegenstand nach Anspruch 1, wobei die zum Körper hin weisende, außen angeordnete Fläche eine flüssigkeitsdurchlässige Abdeckung (32) umfaßt.

7. Der Gegenstand nach Anspruch 1, wobei ein Beschichtungsgewicht des Klebstoffs (18; 46; 401) zwischen ca. 1,5 und ca. 2.000 Milligramm/Quadratzentimeter liegt.

8. Der Gegenstand nach Anspruch 1, wobei der Klebstoff (18; 46; 401) ausgewählt ist aus der Gruppe bestehend aus Lösungsklebstoffen, Emulsionsklebstoffen, Warmschmelzklebstoffen, Heißschmelzklebstoffen und geschäumten Klebstoffen.

9. Der Gegenstand nach Anspruch 1, wobei der Klebstoff (18; 46; 401) hydrophil ist.

10. Ein Verfahren zur Herstellung eines absorptionsfähigen interlabialen Gegenstandes, der so groß und so gestaltet ist, daß er in geeigneter und komfortabler Weise in den Raum zwischen den Schamlippen eindringt, und der umfaßt: eine Höhe, die geeignet ist, um flüssige Ausscheidungen aus dem Körper aufzufangen und zu absorbieren, eine Länge (14), die eine Abdeckung der Vaginaöffnung und der Harnröhrenöffnung gewährleistet, und eine Breite (16), die einen guten Sitz im Raum zwischen den Schamlippen gewährleistet, das folgende Schritte umfaßt:
a) Bereitstellen eines Vlieses aus absorptionsfähigem Material mit einer zentralen Längsachse;
b) Bedecken zumindest eines Teils des Vlieses mit einem flüssigkeitsdurchlässigem Material;
c) Falten des Vlieses auf sich selbst entlang seiner zentralen Längsachse, dadurch Ausbilden eines Falzes und nebeneinander angeordneter Abschnitte;
d) Falten der nebeneinander angeordneten Abschnitte des Vlieses voneinander weg nach außen und koplanar zueinander entlang einer Linie, die bezogen auf den Falz distal ist, dadurch Ausbilden einer Vliesstruktur mit einem Vorsprung und Basisabschnitten, wobei der Abstand zwischen der Linie und dem Falz eine Vorsprungshöhe definiert;
e) Bereitstellen eines flüssigkeitsundurchlässigen Materials mit einer zentralen Längsachse;
f) Falten des flüssigkeitsundurchlässigen Materials auf sich selbst entlang seiner zentralen Längsachse, dadurch Ausbilden eines Falzes und nebeneinander angeordneter Abschnitte;
g) Falten nebeneinander liegender Abschnitte des flüssigkeitsundurchlässigen Materials voneinander weg nach außen und koplanar zueinander entlang einer Linie, die bezogen auf den Falz distal ist, dadurch Ausbilden einer flüssigkeitsundurchlässigen Struktur mit einem Vorsprung und Basisabschnitten, wobei der Abstand zwischen der Faltlinie und dem Falz eine Vorsprungshöhe definiert;
h) Ausrichten der Basisabschnitte des Vlieses und der flüssigkeitsundurchlässigen Basisabschnitte aufeinander und Befestigen beider aneinander, wobei der Vorsprung des Vlieses und der flüssigkeitsundurchlässige Vorsprung außen und in entgegengesetzte Richtungen weisend angeordnet sind, dadurch Ausbilden einer absorptionsfähigen Struktur;
i) Teilen der absorptionsfähigen Struktur in eine Mehrzahl absorptionsfähiger interlabialer Gegenstände; und das weiterhin folgenden Schritt umfaßt: Aufbringen eines Klebstoffs auf zumindest einen Abschnitt einer außen angeordneten Fläche der Basisabschnitte des Vlieses oder einer außen angeordneten Fläche des Vorsprungs des Vlieses.

## Revendications

1. Article interlabial absorbant (20 ; 50 ; 55 ; 60 ; 65 ; 70 ; 75 ; 80 ; 85 ; 90 ; 100 ; 400 ; 500) d'une taille et d'une configuration permettant une pénétration appropriée et confortable dans l'espace entre les lèvres, comprenant : une structure absorbante ayant une partie proximale (2 ; 38 ; 94 ; 102) qui, lors de l'utilisation, fait face au corps et pénètre entre les lèvres, et une partie distale (6 ; 42) qui, lors de l'utilisation, est orientée à l'opposé du corps, comprenant des surfaces faisant face au corps et opposée au corps disposées vers l'extérieur (10, 12, 26, 54) respectivement ; un adhésif (18 ; 46 ; 401) situé sur une partie au moins de la surface opposée au corps disposée vers l'extérieur (10 ; 26) afin d'attacher l'article de manière amovible au corps de l'utilisateur ; une hauteur (13) pour que l'article absorbant (20) pénètre dans l'espace entre les lèvres, et qui permet de capturer et d'absorber les décharges de liquide provenant du corps ; une longueur (14) qui assure la couverture de l'ouverture vaginale et du méat urétral ; et une largeur (16) qui assure une bonne adaptation à l'espace entre les lèvres.

2. Article tel que décrit dans la revendication 1, dans lequel la hauteur (13) se situe dans une plage d'environ 3 mm à environ 6,5 mm.

3. Article tel que décrit dans la revendication 1, dans lequel la hauteur (13) se situe dans une plage d'environ 6,5 mm à environ 9,5 mm.

4. Article tel que décrit dans la revendication 1, dans lequel la surface orientée à l'opposé du corps disposée vers l'extérieur comprend un film arrière imperméable aux liquides.

5. Article tel que décrit dans la revendication 4, dans lequel le film arrière imperméable aux liquides est perméable aux gaz.

6. Article tel que décrit dans la revendication 1, dans lequel la surface faisant face au corps disposée vers l'extérieur comprend un cache perméable aux liquides (32).

7. Article tel que décrit dans la revendication 1, dans lequel le poids de revêtement de l'adhésif (18 ; 46 ; 401) varie d'environ 1, 5 à environ 2000 milligrammes/centimètre carré.

8. Article tel que décrit dans la revendication 1, dans lequel l'adhésif est choisi dans le groupe comprenant des adhésifs en solution, des adhésifs en émulsion, des adhésifs fusibles à moyenne température, des adhésifs fusibles à haute température et des adhésifs en mousse.

9. Article tel que décrit dans la revendication 1, dans lequel l'adhésif (18 ; 46 ; 401) est hydropholique.

10. Procédé de fabrication d'un article interlabial absorbant d'une taille et d'une configuration permettant une pénétration appropriée et confortable dans l'espace entre les lèvres, et comprenant une hauteur permettant de capturer et d'absorber les décharges de liquide provenant du corps, une longueur (14) qui assure la couverture de l'ouverture vaginale et du méat urétral, et une largeur (16) qui assure une bonne adaptation à l'espace entre les lèvres, lequel procédé comprend les étapes suivantes:
a) utiliser un voile d'un matériau absorbant possédant un axe longitudinal central ;
b) recouvrir une partie au moins dudit voile avec un matériau perméable aux liquides ;
c) plier le voile sur lui-même le long de l'axe longitudinal central de manière à former ainsi un bord plié et des sections juxtaposées ;
d) plier les sections juxtaposées du voile vers l'extérieur en les écartant l'une de l'autre et sur un même plan, le long d'une ligne distale au dit bord plié, formant ainsi une structure de voile ayant une protubérance et des sections de base, la distance entre la ligne et le bord plié définissant la hauteur de la protubérance ;
e) utiliser un matériau imperméable aux liquides ayant un axe longitudinal central ;
f) plier le matériau imperméable aux liquides sur lui-même le long de son axe longitudinal central de manière à former ainsi un bord plié et des sections juxtaposées ;
g) plier les sections juxtaposées du matériau imperméable aux liquides vers l'extérieur en les écartant l'une de l'autre et sur un même plan, le long d'une ligne distale au dit bord plié, formant ainsi une structure imperméable aux liquides ayant une protubérance et des sections de base, la distance entre la ligne de pli et le bord plié définissant la hauteur de la protubérance ;
h) aligner et attacher les sections de base du voile aux sections de base imperméables aux liquides, la protubérance du voile et la protubérance imperméable aux liquides étant ainsi disposées vers l'extérieur dans des directions opposées, formant ainsi une structure absorbante ; et
i) séparer la structure absorbante en plusieurs articles interlabiaux absorbants, le procédé comprenant en outre une étape consistant à appliquer un adhésif sur une partie au moins d'une surface disposée vers l'extérieur des sections de base du voile ou d'une surface disposée vers l'extérieur de la protubérance du voile.
